# EUROPÄISCHE PATENTSCHRIFT

(11) **EP 2 528 595 B1**
(45) Veröffentlichungstag und Bekanntmachung des Hinweises auf die Patenterteilung: **05.08.2015**
(21) Anmeldenummer: 11704168.1
(22) Anmeldetag: 28.01.2011
(51) Int. Cl.: A61K 31/12, A61K 31/165, A61K 31/192, A61K 31/198, A61K 31/216, A61P 11/06, A61P 25/00, A61P 11/00, A61P 1/04, A61P 11/02, A61P 15/00, A61P 43/00, A61P 17/00, A61P 9/00, A61P 9/12

(54) **VERBINDUNGEN ZUR VERWENDUNG BEI DER BEHANDLUNG VON ERKRANKUNGEN**
COMPOUNDS FOR USE IN THE TREATMENT OF DISEASES
COMPOSÉS DESTINÉS À ÊTRE UTILISÉS DANS LE TRAITEMENT DE MALADIES

(30) Priorität: 29.01.2010 AT 1222010
(43) Veröffentlichungstag der Anmeldung: 05.12.2012
(60) Teilanmeldung: 15173989.3
(73) Patentinhaber: Inoxia Lifesciences GmbH, 1120 Wien (AT)
(72) Erfinder: KUBIN, Andreas, A-1120 Wien (AT); FURTMÜLLER, Paul, A-1190 Wien (AT); WOLBER, Gerhard, A-1060 Wien (AT); SCHUSTER, Daniela, A-1060 Wien (AT)
(74) Vertreter: Sonn & Partner Patentanwälte
(86) Internationale Anmeldenummer: PCT/AT2011/000050
(87) Internationale Veröffentlichungsnummer: WO 2011/091461

(56) Entgegenhaltungen:
- EP-A2- 0 323 590
- WO-A1-00/15215
- WO-A1-2010/127440
- WO-A2-01/07019
- AU-A1- 2004 242 565
- AU-B2- 774 861
- CN-A- 1 686 297
- US-A- 5 741 926
- US-A- 6 011 000
- US-A1- 2009 215 730
- US-B1- 6 197 743
- PENNOCK G D ET AL: "ISENTIFICATION OF SIMPLE SUBSTITUTED PHEDOLS WITH THYROMIMETIC ACTIVITY: CARDIAC EFFECTS OF 3,5-DIIODO-4-HYDROXYPHENYLPROPIONIC ACID", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, Bd. 268, Nr. 1, 1. Januar 1994 (1994-01-01), Seite 216, XP008063393, ISSN: 0022-3565
- BERI RIPLA ET AL: "Synthesis and antitubercular activity of N-aryl-glycylhydrazides", PROCEEDINGS OF THE INDIAN NATIONAL SCIENCE ACADEMY. PART A.PHYSICAL SCIENCES, INDIAN NATIONAL SCIENCE ACADEMY, NEW DEHLI, IN, Bd. 57, Nr. 5, 1. Januar 1991 (1991-01-01) , Seiten 645-654, XP008137270, ISSN: 0370-0046
- WANG J ET AL: "Role of eosinophil peroxidase in host defense and disease pathology", ARCHIVES OF BIOCHEMISTRY AND BIOPHYSICS, ACADEMIC PRESS, US, Bd. 445, Nr. 2, 15. Januar 2006 (2006-01-15), Seiten 256-260, XP024942973, ISSN: 0003-9861, DOI: DOI:10.1016/J.ABB.2005.10.008 [gefunden am 2006-01-15] in der Anmeldung erwähnt
- WOZEL G: "Eosinophilic dermatoses", DER HAUTARZT ; ZEITSCHRIFT FÜR DERMATOLOGIE, VENEROLOGIE UND VERWANDTE GEBIETE, SPRINGER, BERLIN, DE, Bd. 58, Nr. 4, 1. April 2007 (2007-04-01), Seiten 347-360, XP019499634, ISSN: 1432-1173, DOI: DOI:10.1007/S00105-007-1318-9 in der Anmeldung erwähnt

## Beschreibung

Die gegenständliche Erfindung betrifft Verbindungen zur Behandlung von inflammatorischen Erkrankungen, die in Zusammenhang mit Eosinophiler Peroxidase stehen, und wobei die inflammatorische Erkrankung ausgewählt ist aus der Gruppe bestehend aus Asthma bronchiale, cystische Fibrose, Rhinitis, Sinusitis und Churg-Strauss Syndrom.

Humane Enzyme der Klasse der Peroxidasen sind Teil der unspezifischen Immunabwehr. Sie werden bei der Abwehr von pathogenen Mikroorganismen in hohen Konzentrationen freigesetzt und katalysieren diverse Oxidationsreaktionen von Biomolekülen, wodurch Eindringlinge, wie Bakterien und Viren, inaktiviert werden. Oftmals kommt es dabei aber durch eine Überproduktion dieser Proteine auch zu oxidativen Schädigungen körpereigener Gewebe und Entzündungen sind die Folge.

Diese Enzyme werden daher mit vielen Krankheiten in Zusammenhang gebracht, die in unserem Kulturkreis eine bedeutende Rolle spielen. Das sind sogenannte "autoenzyminduzierte" Erkrankungen, wobei insbesondere die körpereigenen Proteine MPO (Myeloperoxidase) und EPO (Eosinophile Peroxidase; EC Nummer: 1.11.1.7) mit der Pathogenese vieler entzündlicher Krankheiten in Verbindung gebracht werden (siehe Tabelle 1). Zusätzlich enthält Milch Laktoperoxidase (LPO), welche antimikrobielle und antioxidative Eigenschaften aufweist.

**Tabelle 1: Beispiele für "autoenzyminduzierte" Erkrankungen, an deren Verlauf Peroxidasen durch Überproduktion beteiligt sind (siehe auch Davies, MJ. et al. Antioxidants & Redox Signaling 10 (2008) 1199 - 1234).**

| ***Krankheit*** | ***Enzym*** |
|---|---|
| Asthma (chronisch) | EPO |
| Raucherlunge (COPD) | MPO |
| Alzheimer | MPO |
| Multiple Sklerose (MS) | MPO, EPO |
| Artheriosklerose | MPO |
| Cystische Fibrose | EPO |
| Colitis ulcerosis | EPO |
| Mastidien (vet. Med.) | LPO |
| Krebs (nach Infektionen) | EPO |
| Bluthochdruck (NO-Signal) | EPO |

Deshalb ist es von Vorteil gegen MPO und EPO, die prominentesten und aggressivsten Vertreter dieser Enzymklasse, spezifische Hemmstoffe (Inhibitoren) zu entwickeln, die in der Folge als Basis für neue Medikamente und Therapien für inflammatorische Erkrankungen dienen.

Die EPO gilt als Hauptursache für viele Erkrankungen, insbesondere den chronischen Verlauf von Asthma bronchiale. Durch einen gut verträglichen Inhibitor könnte erstmals ein echter Heilungsansatz für chronisches Asthma bronchiale zur Verfügung gestellt werden. Ähnliches gilt für Cystische Fibrose und andere inflammatorische Prozesse, bei welchen EPO als Hauptverursacher beteiligt ist. Diese schwerwiegenden und in der westlichen Welt stark zunehmenden Erkrankungen zeigen meist einen chronischen Verlauf und konnten bisher nur mit sehr geringem Erfolg therapiert werden.

Das körpereigene Protein Eosinophile Peroxidase (EPO) wird ausgeschüttet, sobald Eosinophile Blutkörperchen (weiße Blutzellen, d.h. Leukozyten) stimuliert werden (z.B. beim Eindringen von pathogenen Substanzen oder Parasiten, d.h. Infektionen). Gleichzeitig kommt es am membrangebundenen NADHP-Oxidase-Komplex zu einer erhöhten Aufnahme von Sauerstoff ins Phagosom ("Respiratory Burst"), wodurch eine Reihe reaktiver Sauerstoffspezien (vor allem Superoxid) freigesetzt werden. Diese werden in der Folge zu Wasserstoffperoxid (H₂O₂) dismutiert und durch Eosinophile Peroxidase zu Wasser reduziert (Mitra, SN. et al. Redox Report 5 (2000) 215-224).

Durch dieses EPO/H₂O₂-System kommt einerseits die physiologische Rolle des Enzyms zum Tragen (Abwehr von Pathogenen), andererseits verursacht es unspezifische und spezifische Zellschädigungen.

Zu den unspezifischen Gewebeschädigungen gehören die Zerstörung von Zellen/Zellwänden, da EPO aufgrund der sehr hohen positiven Ladung (pI > 11) imstande ist, die Lipidmembran von Zellen zu durchdringen. EPO zerstört daher auf dem Weg zu den Zielorten der Infektion Zellen sowie Gewebe und ruft dadurch Entzündungen hervor.

Außerdem tragen Eosinophile Zellen zur Pathogenese von Allergen-gesteuerten Erkrankungen bei, wie Asthma bronchiale. Von Asthma bronchiale spricht man bei Entzündung bzw. erhöhter Empfindlichkeit der Schleimhäute der Bronchien, die zur Verengung der Atemwege führt. Diesem Krankheitsbild liegt die Stimulation bestimmter Abwehrzellen, sogenannter Mastzellen, über Zytokine, wie Interleukin 5 (IL 5), zugrunde. Im Fall von Asthma werden Mastzellen und Eosinophile Granulozyten im Bronchialbereich angelockt. Diese Zellen setzen Substanzen (vor allem Histamin) frei, die unter anderem die Atemwegsmuskulatur zusammenziehen und die Schleimproduktion in den Lungen anregen. Diese Reaktion findet meist sehr schnell statt, innerhalb von 15 bis 30 Minuten nach Kontakt mit dem auslösenden Stoff und/oder Stress. Später (innerhalb von zwei bis vier Stunden) wandern dann Entzündungszellen (Eosinophile Granulozyten) in die Wände der Bronchien ein und lösen dort die chronische Verlaufsform (Entzündung) aus. Werden diese Zellen stimuliert, setzen sie cytotoxische Proteine frei, die viele der pathologischen Charakteristika von Asthma fördern: die Denaturierung des Lungen-Epithels, die Zerstörung der Epithel-Morphologie, erhöhte mikrovaskulare Permeabilität und Ödeme. Bei der Entstehung einer chronischen Entzündung werden aber ebenso Moleküle freigesetzt, die in das "Remodelling" (Wiederherstellung) von Gewebe involviert sind. Dadurch wird zerstörtes Gewebe wieder nachgebildet und die Einlagerung von "unelastischem" Bindegewebe verhindert.

Spezifische Zellschädigungen werden durch eine Reihe von aggressiven Oxidationsprodukten von EPO und diffusionsfähigen freien Radikalen hervorgerufen, die im enzymatischen Reaktionssystem EPO/H₂O₂ produziert werden. Aufgrund des außergewöhnlichen Redoxpotentials einer Enzymzwischenstufe (Compound I) ist EPO in der Lage, diverse kleine Moleküle zu oxidieren. Zu diesen physiologisch relevanten Enzymsubstraten gehören Nitrit (NO₂⁻), Bromid (Br⁻) sowie das Pseudohalogenid Thiocyanat (SCN⁻). Es entstehen in der Folge hochreaktive Substanzen, wie Stickstoffdioxid-Radikale (NO₂^{·}), Hypobromit (⁻OBr) sowie Hypothiocyanat (⁻OSCN) bzw. Cyanat (⁻OCN). Es ist weiters darauf hinzuweisen, dass die biologischen Konsequenzen des EPO/H₂O₂-Systems stark substratspezifisch sind. So ist die physiologische Serumkonzentration von SCN⁻ wesentlich höher (bzw. kann diätetisch günstig beeinflusst werden) als jene von Br⁻ oder NO₂⁻. Damit aktiviert beispielsweise das Oxidationsprodukt ⁻OSCN den Transkriptionsfaktor NF-κB wesentlich stärker als NO₂^{·} und wirkt daher pro-inflammatorischer im MAP-Kinase-System (Wang, J. et al. Arch Biochem Biophys 445 (2006) 256-260). Diese hochaktiven Reaktionsprodukte fungieren nun einerseits als Teil der passiven Immunabwehr und greifen in den Körper eingedrungene große Parasiten an, wodurch sie der physiologische Rolle der EPO nachkommen.

Andererseits können diese Substanzen in nicht-enzymatischen Reaktionen große Biomoleküle (z.B. Lipide, Proteine, DNA, RNA) angreifen, wodurch diese in ihrer Struktur und/oder Funktionalität verändert werden. Es kommt zum Einbau von Brom- oder Nitrogruppen, speziell an Hydroxy- und Aminogruppen (Bromo- und Nitrotyrosine, Bromohydrine, Bromoaldehyde, Bromonucleotide, Lipidperoxide). So konnten beispielsweise im Sputum von Asthmapatienten 3-Bromotyrosine (Biomarker) nachgewiesen werden (Aldridge, CJ. et al. Free Radical Biology & Medicine 33 (2002) 6, 847-856).

In anderen Fällen konnte eine signifikante Übereinstimmung von chronischen Infektionen/Entzündungen und der Pathogenese von Krebs nachgewiesen werden, welche auf oxidative Schäden an der DNA zurückgeführt werden kann (z.B. *Schistosoma haematobium* und Blasenkrebs, oder *Opisthorcis vicerrini* und Cholangiokarzinom (Gallengangkrebs) (Mitra, SN. et al. Redox Report 5 (2000) 215-224).

Weiters ist EPO an der Biochemie der vasoaktiven, d.h. blutgefäßerweiternden, Substanz Stickstoffmonoxid (NO) beteiligt, das eine wesentliche Rolle bei der Angiogenese, der Regulation des Blutdrucks, der Erweiterung der Bronchien (z.B. bei Neugeborenen) sowie anderer physiologischer Phänomene spielt. Es wird vermutet, dass NO von EPO Compound I und Compound II oxidiert, als NO⁺ freigesetzt wird und mit Superoxid zu Peroxynitrit (ONOO⁻ ) reagiert. Diese hochreaktive Verbindung (ein Marker für oxidativen Stress) greift wiederum Lipide und Proteine an, wodurch Nitrotyrosine und Lipidperoxide entstehen. Andererseits steht durch das Abfangen von NO dieses wichtige regulatorische zweiatomige Signalmolekül nicht mehr zur Verfügung, wodurch wichtige biologische Funktionen (z.B. als Botenstoff) nicht mehr oder nur zum Teil erfüllt werden können (Abu-Soud, HM. et al. Biochem 40 (2001) 11866-11875).

Treten derartige Symptome auf, ist nachgewiesen, dass die Plasma- beziehungsweise Gewebskonzentration an Eosinophiler Peroxidase beziehungsweise deren "Fingerprint" an Reaktionsprodukten (z.B. bromierte Lipide und Proteine) mit dem Grad der Erkrankung korreliert. Sowohl Eosinophile Blutzellen als auch Eosinophile Peroxidase sind in Blut, Sputum, bronchialem Gewebe und der Bronchoalveolar-Spülung von Asthmatikern zu finden und dienen der Medizin heute als direkter, quantifizierbarer Marker von Asthma sowie als indirekter Indikator einer Entzündung und das Ansprechen eines Patienten auf Asthma-Therapien.

In der WO 2008/121670 werden Pyrimidinylhydrazide und deren Verwendung in der Behandlung von Asthma bronchiale beschrieben.

In der WO 00/073280 werden Catechin-substituierte Hydrazone und deren Verwendung in der Behandlung von Asthma bronchiale beschrieben.

Die WO 2009/145360 betrifft Phenyl- bzw. Thiophenderivate, welche ebenfalls zur Behandlung von Asthma bronchiale eingesetzt werden können.

Die WO 2004/080377 offenbart Phenylhydrazide, welche geeignet sind, Kaliumkanäle in Zellen zu modulieren, wodurch unter anderem Krankheiten wie Asthma bronchiale behandelt werden können.

In der US 2003/0225102 und der WO 2002/006224 werden Hydrazide beschrieben, welche mit einem heterozyklischen Substituenten substituiert sind. Diese Verbindungen können zur Behandlung von Asthma bronchiale eingesetzt werden.

In der WO 2007/026215, der WO 2005/123688, der DE 10 2006 005 179, der US 5,571,846, der EP 0 323 590, der WO 01/032156, der WO 2005/085185 und der US 4,082,846 werden Verbindungen mit einer Hydrazinstruktur beschrieben, welche geeignet sind bei der Behandlung von verschiedensten Krankheiten eingesetzt zu werden.

In der WO 2001/07019 wird die Verwendung von N-AcetylCystein bei der Herstellung von topischen pharmazeutischen Zusammensetzung für die Behandlung von allergischen Erkrankungen der Atemwege geoffenbart Gegenstände, die nicht durch den Schutzumfang der Ansprüche umfasst sind, sind nicht Teil der beanspruchten Erfindung.

Es ist eine Aufgabe der vorliegenden Erfindung Verbindungen zur Verfügung zu stellen, welche in der Lage sind die Aktivität von Eosinophiler Peroxidase signifikant oder zur Gänze zu inhibieren.

Es hat sich überraschender Weise herausgestellt, dass bestimmte Verbindungen in der Lage sind die Aktivität von Eosinophiler Peroxidase zu inhibieren. Daher betrifft die vorliegende Erfindung Verbindungen mit der allgemeinen Formel (III): 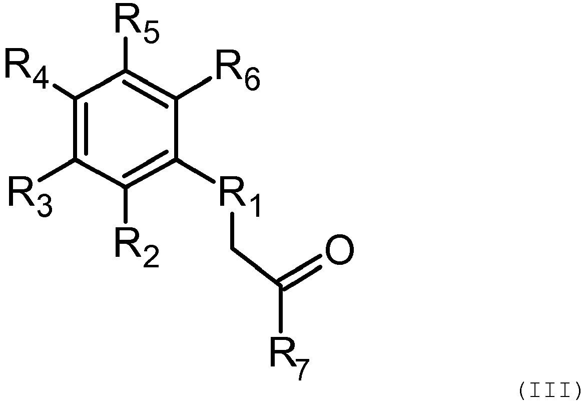 zur Verwendung bei der Behandlung und/oder Prävention von inflammatorischen Erkrankungen, welche in Zusammenhang mit Eosinophiler Peroxidase stehen, wobei
R₁ NH ist,
R₂, R₃, R₄, R₅ und R₆ unabhängig voneinander H, OH, F, Cl, Br, I oder eine C₁ bis C₅ Alkylgruppe sind und
R₇ H, OH, NH₂, NH-NH₂ oder CH₃ ist, und wobei die inflammatorische Erkrankung ausgewählt ist aus der Gruppe bestehend aus Asthma bronchiale, cystische Fibrose, Rhinitis, Sinusitis und Churg-Strauss Syndrom.

Die vorliegende Erfindung geht aus von Hydraziden mit der allgemeinen Formel (I): 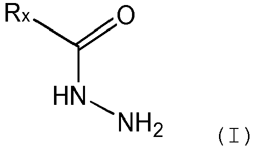 zur Behandlung und/oder Prävention von Erkrankungen, insbesondere inflammatorischen Erkrankungen, welche in Zusammenhang mit Eosinophiler Peroxidase stehen, wobei R_{X} eine heterozyklische Verbindung (heterozyklischer Rest), wie Pyridin, Indol, Pyrazol oder Pyrimidin, oder eine aromatische Verbindung (aromatischer Rest), wie Naphthol, Benzol oder Phenylaminoethan, ist.

Für die inhibitorische Aktivität der erfindungsgemäßen Verbindungen ist die freie terminale Aminogruppe von Vorteil, die als Elektronenakzeptor fungiert.

Darüber hinaus sind aber noch sterische und/oder elektrochemische Eigenschaften dieser Verbindung für die Bindung und/oder enzymatische Reaktion dieser Verbindungen mit EPO verantwortlich. Ein Pharmakophormodell hat gezeigt, dass die erfindungsgemäßigen Substanzen verschiedene Motive (z.B. Wasserstoffbrücken-Donoren, Wasserstoffbrücken-Akzeptoren, aromatische Ringe/Bereiche, hydrophobe Bereiche) aufweisen müssen. Daraus ergibt sich folgende beispielhafte Struktur, die auch Bindungslängen und Domänen berücksichtigt (II):

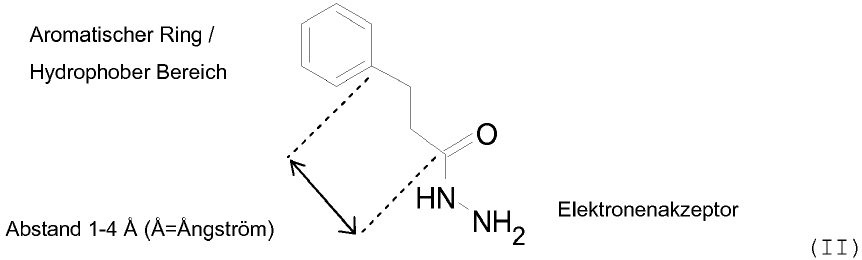

Die erfindungsgemäß bevorzugten Phenyl-Amino-Ethan-Hydrazide (PAEH's) und deren Derivate entsprechen diesem Modell, wobei der Abstand zwischen Benzolring und Säurehydrazidgruppe in diesem Fall 2,65 Å beträgt (IIIa):

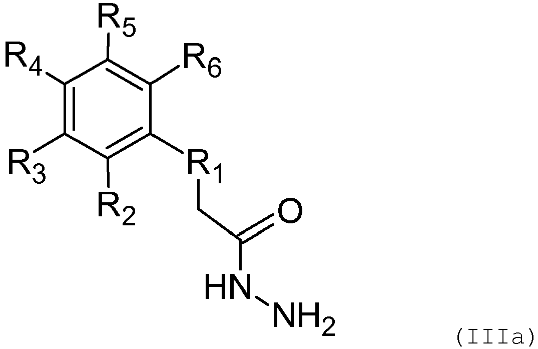

Substituent R₁ ist NH und die Substituenten R₂, R₃, R₄, R₅ und R₆ sind unabhängig voneinander H, OH, F, Cl, Br, I oder eine C₁ bis C₅ Alkylgruppe.

Eine zentrale Schlüsselrolle bei der Produktion der aggressiven, zellschädigenden Substanzen spielt - wie bereits eingangs diskutiert - die Eosinophile Peroxidase, EPO. Diese Prozesse, insbesondere inflammatorische Prozesse, bei denen die EPO beteiligt ist, können durch die Verwendung der erfindungsgemäßen Substanzen inhibiert werden, so dass Erkrankungen, die in Zusammenhang mit Eosinophiler Peroxidase stehen, behandelt werden können.

Die erfindungsgemäßen Verbindungen sind selektiv für Eosinophile Peroxidase (Vorkommen in weißen Blutzellen) und die homologe Laktoperoxidase (Vorkommen in Muttermilch und im Speichel). Diese Verbindungen sind jedoch nicht in der Lage Myeloperoxidase, insbesondere humane Myeloperoxidase, im gleichen Ausmaß zu inhibieren, was den gezielten Einsatz dieser Verbindungen als spezifisches Medikament, selektiv gegen EPO, ermöglicht.

Auf Grund der starken inhibitorischen Wirkung der erfindungsgemäßen Substanzen ist es nämlich möglich, therapeutische Anwendungen mit sehr niedrigen Dosierungen zu entwickeln. Dabei können lokale oder systemische Konzentrationen von etwa 0,001 bis 10 µM ausreichend sein.

Die erfindungsgemäßen Verbindungen sind in der Fachwelt hinreichend bekannt und werden nach bekannten Verfahren hergestellt (siehe z.B. Finger, GC. et al. J Am Chem Soc 81 (1959) 94-101). Die meisten N-Arylglycine werden ebenso wie ihre Ester, Hydrazide und andere Derivate zur biologischen Überprüfung ihres tuberkulostatischen Potentials hergestellt. p-Alkylaniline und p-Cyclohexylaniline werden mittels Beckmann Umlagerung von Oximen der korrespondierenden p-substituierten Acetophenone hergestellt. p-Alkoxyaniline werden über Alkylierung von p-Benzalaminophenol mit Alkyl-Halogeniden und NaOH in wässrigem Ethanol hergestellt mit nachfolgender Hydrolyse der Aldimine mit HCl. (Tien, NB. et al. Org Chem 23 (1958) 186-8).

Der Begriff "inflammatorische Erkrankungen, welche in Zusammenhang mit Eosinophiler Peroxidase stehen" bezieht sich auf Krankheiten und Krankheitszustände, die auf eine erhöhte Aktivität der EPO in einem Individuum zurückzuführen sind (siehe z.B. Davies, MJ. et al. Antioxidants & Redox Signaling 10 (2008) 1199 - 1234; Wang, J. et al. Arch Biochem Biophys 445 (2006) 256-260; Mitra, SN. et al. Redox Report 5 (2000) 215-224). Derartige Erkrankungen sind dem Fachmann durchaus bekannt, wie dies auch eingangs diskutiert ist. Der Zusammenhang zwischen der EPO-Aktivität und Krankheiten, die Folge der EPO-Aktivität sind, ist ebenfalls in der Fachwelt hinreichend bekannt. Beispielsweise konnten im Sputum von Patienten, die an Asthma bronchiale leiden, 3-Bromotyrosine (Biomarker) mittels GC-MS (Gaschromatographie-Massenspektroskopie) nachgewiesen werden, die durch Modifikation von Proteinen mittels ⁻OBr, einem EPO-Oxidationsprodukt, entstanden sind (Aldridge, CJ. et al. Free Radical Biology & Medicine 33 (2002) 847-856).

Hypothiocyanat (⁻OSCN) bzw. NO₂^{·}, Reaktionsprodukte der EPO, aktivieren den Transkriptionsfaktor NF-κB und wirken daher im MAP-Kinase-System pro-inflammatorisch. Transgene Mäuse (EPOknock-out) zeigten wesentlich geringere Schädigungen durch Colitis ulceros. Das gilt auch für andere chronische Entzündungen wie Morbus Crohn oder Cystische Fibrose (Wang, J. et al. Arch Biochem Biophys 445 (2006) 256-260).

Auch Tumorerkrankungen können Folge einer erhöhten EPO-Aktivität sein, da diese zur oxidativen Schädigung der DNA führt, die durch reaktive Sauerstoffspezien (z.B. Bromonucleotide, Singulett-Sauerstoff) nach Infektionen hervorgerufen wird (z.B. *Schistosoma haematobium* und Blasenkrebs, oder *Opisthorcis vicerrini* und Cholangiokarzinom (Gallengangkrebs) (Mitra et al. Redox Report 5 (2000) 215-224). Eine alternative Bezeichnung für "inflammatorische Erkrankungen, welche in Zusammenhang mit Eosinophiler Peroxidase stehen" sind inflammatorische Erkrankungen, die auf einer erhöhte Aktivität der EPO im Körper beruhen, wobei die erhöhte Aktivität sich auf ein Durchschnittsindividuum bezieht, welches keine Erkrankungen aufweist, die eine Folge erhöhter EPO-Aktivität darstellen.

Durch Migration von EPO bzw. reaktiver Oxidationsprodukte (⁻ OBr bzw. NO₂^{·}) werden Lipiddoppelschichten sowie Membranproteine und Zellwände modifiziert (Bromo- und Nitrotyrosine, LipidPeroxide), desintegriert und letztendlich zerstört (Wang, J. et al. Arch Biochem Biophys 445 (2006) 256-260). Dies führt zu Gewebsschädigungen und Nekrosen. Durch den Einsatz der selektiven Inhibitoren wird die gewebszerstörende Wirkung der EPO verhindert und gleichzeitig aber die gewebsbildende Funktion der Eosinophile Granulozyten aufrechterhalten. Somit kann z.B. der bislang irreversible und chronische Verlauf von Asthma bronchiale (EPO-Inhibitor) gestoppt werden und sogar ein Heilungsansatz durch diese neue Arzneimittelgruppe gegeben sein.

Die erfindungsgemäßen Verbindungen umfassen u.a. pharmazeutisch verträgliche Säureadditionssalze, unter denen erfindungsgemäß solche Salze zu verstehen sind, die ausgewählt sind aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure, wobei die Salze der Salzsäure, Bromwasserstoffsäure, Schwefelsäure, Phosphorsäure, und Essigsäure besonders bevorzugt sind.

Es hat sich gezeigt, dass es von Vorteil ist wenn R₇ eine freie Aminogruppe, vorzugsweise eine Hydrazidgruppe, aufweist. Die Aminogruppen derartiger Verbindungen mit den allgemeinen Formeln (I) bzw. (IIIa) und (IV) sind für dessen Wirkung als EPO-Inhibitor von Vorteil. D.h. die erfindungsgemäßen Verbindungen sollten am Wirkort die freie Aminogruppe aufweisen. Es ist jedoch möglich, um die Verträglichkeit der erfindungsgemäßen Verbindungen zu erhöhen die Aminogruppe mit einer Schutzgruppe zu versehen, die gegebenenfalls am Wirkort entfernt wird (Prodrug-Konzept). Selbstverständlich kann R₇ der Verbindung mit der allgemeinen Formel (III) auch ein H, OH oder CH₃ Rest sein. Auch derartige Verbindungen sind in der Lage eosinophile Peroxidase mit hoher Wirksamkeit zu inhibieren.

Gemäß einer besonders bevorzugten Ausführungsform der vorliegenden Erfindung ist R₁ NH, wobei das Hydrazid die allgemeine Formel (IV) aufweist:

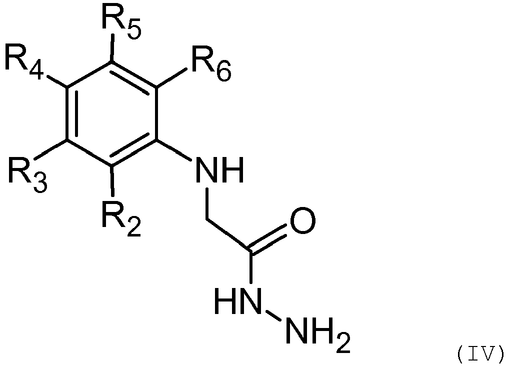

Gemäß einer bevorzugten Ausführungsform der vorliegenden Erfindung ist die C₁ bis C₅ Alkylgruppe ausgewählt aus der Gruppe bestehend aus CH₃ und CH₂CH₃.

Gemäß einer weiteren bevorzugten Ausführungsform der vorliegenden Erfindung ist R₁ NH, R₂ F oder H, R₃ Cl, Br oder H, R₄ Cl, F, CH₃ oder H, R₅ und R₆ H und R₇ OH oder NH-NH₂.

Gemäß einer besonders bevorzugten Ausführungsform der vorliegenden Erfindung weist die erfindungsgemäße Verbindung (III) die folgenden Substituenten auf (siehe Tabelle A):

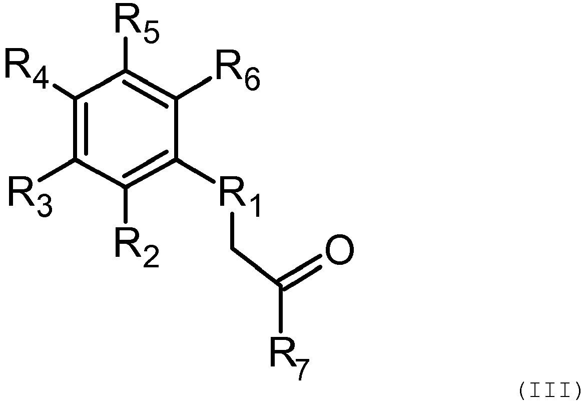

**Tabelle A:**

| **Nr.** | **R₁** | **R₂** | **R₃** | **R₄** | **R₅** | **R₆** | **R₇** |
|---|---|---|---|---|---|---|---|
| 1 | NH | H | H | F | H | H | NH-NH₂ |
| 2 | NH | H | H | Cl | H | H | NH-NH₂ |
| 3 | NH | F | H | H | H | H | NH-NH₂ |
| 4 | NH | H | Cl | F | H | H | NH-NH₂ |
| 5 | NH | H | Br | H | H | H | NH-NH₂ |
| 6 | NH | H | H | H | H | H | NH-NH₂ |
| 7 | NH | F | H | F | H | H | NH-NH₂ |
| 8 | NH | H | H | CH₃ | H | H | NH-NH₂ |
| 9 | NH | F | Cl | H | H | H | NH-NH₂ |
| 10 | NH | Cl | H | H | H | H | NH-NH₂ |
| 11 | NH | F | H | H | Cl | H | NH-NH₂ |
| 12 | NH | F | H | Cl | H | H | NH-NH₂ |
| 13 | NH | F | H | H | H | Cl | NH-NH₂ |
| 14 | NH | H | Cl | H | H | H | NH-NH₂ |
| 15 | NH | H | F | H | H | H | NH-NH₂ |
| 16 | NH | F | F | H | H | H | NH-NH₂ |
| 17 | NH | H | F | F | H | H | NH-NH₂ |
| 18 | NH | H | F | H | F | H | NH-NH₂ |
| 19 | NH | H | F | H | H | F | NH-NH₂ |
| 20 | NH | CH₃ | H | H | H | H | NH-NH₂ |
| 21 | NH | H | CH3 | H | H | H | NH-NH₂ |
| 22 | NH | H | H | H | CH₃ | H | NH-NH₂ |
| 23 | NH | H | H | H | H | CH3 | NH-NH₂ |
| 24 | NH | CH₂CH₃ | H | H | H | H | NH-NH₂ |
| 25 | NH | H | CH₂CH₃ | H | H | H | NH-NH₂ |
| 26 | NH | H | H | CH₂CH₃ | H | H | NH-NH₂ |
| 27 | NH | H | H | H | CH₂CH₃ | H | NH-NH₂ |
| 28 | NH | H | H | H | H | CH₂CH₃ | NH-NH₂ |
| 29 | NH | F | H | CH3 | H | H | NH-NH₂ |
| 30 | NH | F | CH₃ | H | H | H | NH-NH₂ |
| 31 | NH | F | Br | H | H | H | NH-NH₂ |
| 32 | NH | F | H | Br | H | H | NH-NH₂ |
| 33 | NH | F | H | H | Br | H | NH-NH₂ |
| 34 | NH | F | H | H | H | Br | NH-NH₂ |
| 35 | NH | H | F | H | Br | H | NH-NH₂ |
| 36 | NH | H | F | Br | H | H | NH-NH₂ |
| 37 | NH | Br | F | H | H | H | NH-NH₂ |
| 38 | NH | Br | H | Cl | H | H | NH-NH₂ |
| 39 | NH | Br | H | H | Cl | H | NH-NH₂ |
| 40 | NH | Br | Cl | H | H | H | NH-NH₂ |
| 41 | NH | Br | H | H | H | Cl | NH-NH₂ |
| 42 | NH | H | Br | F | H | H | NH-NH₂ |
| 43 | NH | H | Br | H | F | H | NH-NH₂ |
| 44 | NH | H | Br | Cl | H | H | NH-NH₂ |
| 45 | NH | H | Br | H | Cl | H | NH-NH₂ |
| 46 | NH | H | Br | H | H | Cl | NH-NH₂ |
| 47 | NH | Cl | H | H | F | H | NH-NH₂ |
| 48 | NH | Cl | F | H | H | H | NH-NH₂ |
| 49 | NH | Cl | H | F | H | H | NH-NH₂ |
| 50 | NH | Cl | H | H | F | H | NH-NH₂ |
| 51 | NH | Cl | H | H | H | F | NH-NH₂ |
| 52 | NH | H | Cl | F | H | H | NH-NH₂ |
| 53 | NH | H | Cl | H | F | H | NH-NH₂ |
| 54 | NH | H | Cl | H | H | F | NH-NH₂ |
| 55 | NH | H | H | Cl | F | H | NH-NH₂ |
| 56 | NH | H | H | Cl | H | F | NH-NH₂ |
| 57 | NH | H | H | H | H | F | NH-NH₂ |
| 169 | NH | H | H | F | H | H | OH |
| 170 | NH | H | H | Cl | H | H | OH |
| 171 | NH | F | H | H | H | H | OH |
| 172 | NH | H | Cl | F | H | H | OH |
| 173 | NH | H | Br | H | H | H | OH |
| 174 | NH | H | H | H | H | H | OH |
| 175 | NH | F | H | F | H | H | OH |
| 176 | NH | H | H | CH₃ | H | H | OH |
| 177 | NH | F | Cl | H | H | H | OH |
| 178 | NH | Cl | H | H | H | H | OH |
| 179 | NH | F | H | H | Cl | H | OH |
| 180 | NH | F | H | Cl | H | H | OH |
| 181 | NH | F | H | H | H | Cl | OH |
| 182 | NH | H | Cl | H | H | H | OH |
| 183 | NH | H | F | H | H | H | OH |
| 184 | NH | F | F | H | H | H | OH |
| 185 | NH | H | F | F | H | H | OH |
| 186 | NH | H | F | H | F | H | OH |
| 187 | NH | H | F | H | H | F | OH |
| 188 | NH | CH₃ | H | H | H | H | OH |
| 189 | NH | H | CH₃ | H | H | H | OH |
| 190 | NH | H | H | H | CH₃ | H | OH |
| 191 | NH | H | H | H | H | CH₃ | OH |
| 192 | NH | CH₂CH₃ | H | H | H | H | OH |
| 193 | NH | H | CH₂CH₃ | H | H | H | OH |
| 194 | NH | H | H | CH₂CH₃ | H | H | OH |
| 195 | NH | H | H | H | CH₂CH₃ | H | OH |
| 196 | NH | H | H | H | H | CH₂CH₃ | OH |
| 197 | NH | F | H | CH₃ | H | H | OH |
| 198 | NH | F | CH3 | H | H | H | OH |
| 199 | NH | F | Br | H | H | H | OH |
| 200 | NH | F | H | Br | H | H | OH |
| 201 | NH | F | H | H | Br | H | OH |
| 202 | NH | F | H | H | H | Br | OH |
| 203 | NH | H | F | H | Br | H | OH |
| 204 | NH | H | F | Br | H | H | OH |
| 205 | NH | Br | F | H | H | H | OH |
| 206 | NH | Br | H | Cl | H | H | OH |
| 207 | NH | Br | H | H | Cl | H | OH |
| 208 | NH | Br | Cl | H | H | H | OH |
| 209 | NH | Br | H | H | H | Cl | OH |
| 210 | NH | H | Br | F | H | H | OH |
| 211 | NH | H | Br | H | F | H | OH |
| 212 | NH | H | Br | Cl | H | H | OH |
| 213 | NH | H | Br | H | Cl | H | OH |
| 214 | NH | H | Br | H | H | Cl | OH |
| 215 | NH | Cl | H | H | F | H | OH |
| 216 | NH | Cl | F | H | H | H | OH |
| 217 | NH | Cl | H | F | H | H | OH |
| 218 | NH | Cl | H | H | F | H | OH |
| 219 | NH | Cl | H | H | H | F | OH |
| 220 | NH | H | Cl | F | H | H | OH |
| 221 | NH | H | Cl | H | F | H | OH |
| 222 | NH | H | Cl | H | H | F | OH |
| 223 | NH | H | H | Cl | F | H | OH |
| 224 | NH | H | H | Cl | H | F | OH |
| 225 | NH | H | H | H | H | F | OH |

Gemäß einer bevorzugten Ausführungsform der vorliegenden Erfindung ist die Verbindung ausgewählt aus der Gruppe bestehend aus 2-Fluoro-Phenylaminoethan-Hydrazid, 4-Fluoro-Phenylaminoethan-Hydrazid, 2,4-di-Fluoro-Phenylaminoethan-Hydrazid, 4-Chloro-Phenylaminoethan-Hydrazid, 3-Chloro-4-Fluoro-Phenylaminoethan-Hydrazid, 3-Bromo-4-Fluoro-Phenylaminoethan-Hydrazid, 4-Methyl-Phenylaminoethan-Hydrazid, Phenylaminoethan-Hydrazid, N-(2-Fluorophenyl)Glycin und 2-[(4-chlorophenyl)amino]Essigsäure.

Mit den erfindungsgemäßen Verbindungen können inflammatorische Erkrankungen behandelt werden, deren Ursache in einer überhöhten EPO-Aktivität zu finden ist. Die Eosinophilen Granulozyten und die EPO sind Bestandteil der unspezifischen Immunabwehr. Besonders bei inflammatorischen Prozessen kommt es zu Anhäufungen dieser weißen Blutkörperchen, die auch chronische Entzündungen hervorrufen können. Die inflammatorische Erkrankung ist dabei ausgewählt aus der Gruppe bestehend aus Asthma bronchiale, cystische Fibrose, Rhinitis, Sinusitis und Churg-Strauss Syndrom.

Übersicht beispielhafter Erkrankungen die durch die Eosinophile Peroxidase (EPO) verursacht werden, beziehungsweise an deren Verlauf die EPO beteiligt ist:

| **Erkrankung** | **Entität** | **Literatur** |
|---|---|---|
| Asthma bronchiale | Chronische inflammatorische Erkrankung der Atemwege, Allergie | (1), (4), (7), (15) |
| Sinusitis | Chronische Entzündung der Nebenhöhlen/Nasenkatarrh, Schnupfen | (9), (10) |
| Rhinitis | Nasenkatarrh, Schnupfen | (15), (16) |
| Cystische Fibrose | Genetisch bedingt Atemwegser-krankung | (1) |
| Churg-Strauß Syndrom | Entzündung der kleinen Blutgefäße: Krankheitsbild von Rhinitis/Asthma | (14) |

| | | |
|---|---|---|
| (1) Davies MJ, et al. Antioxidants & Redox Signaling. 10, 2008: 1199 - 1234. (4) Mitra SN, et al. Redox Rep. 5, 2000: 215 - 224. (7) Heinecke JW. J Clin Invest. 105, 2000: 1331 - 1332. (9) Bernardes JF, et al. Otolaryngol Head Neck Surg. 131, 2004:69-703. (10) Bachert C, et al. Acta Otorhinolaryngol Belg. 51, 1997:209-217. (14) Eustace JA, et al. J Am Soc Nephrol 10, 1999: 2048 - 2055. (15) Janeway's Immunobiology, ISBN 0-8153-4123-7, Garland Science, Taylor & Francis Group, 2008, 7th Edition: 566 - 583. (16) Nielsen LP, et al. Allergy 64, 2009: 733 - 337. | | |

In verschiedenen entzündeten Organen und Geweben sowie daraus gewonnenen Sekreten konnte EPO und/oder deren Reaktionsprodukte (z.B. nitrierte, bromierte Lipide, Proteine, DNA) nachgewiesen werden. Das belegt einerseits die passive Immunantwort durch EPO im Rahmen der Phagozytose, andererseits zeigt sich auch massiv die gewebszerstörende Wirkung der EPO und deren Reaktionsprodukten. Beispielsweise konnte im Sputum von Asthmapatienten EPO radioimmunologisch nachgewiesen werden sowie 3-Bromotyrosin mittels Gaschromatographie-Massenspektroskopie (GC-MS) (Aldridge et al. Free Radical Biology & Medicine 33 (2002) 847-856).

Auch bei allergischen Erkrankungen wie Rhinitis (Nasenschleimhautentzündung) ist EPO entscheidend beteiligt (Hrdlickova, B. et al. Int Arch Allergy Immunol. 150 (2009) 184-91).

Die erfindungsgemäßen Verbindungen können auf unterschiedliche Weise verabreicht werden. Je nach Erkrankung können die Verbindungen systemisch oder lokal verabreicht werden. Die erfindungsgemäßen Verbindungen, insbesondere Phenyl-Amino-Ethan-Hydrazid (PAEH) bzw. dessen Derivate, sind daher vorzugsweise in einer intravenösen, intrakavitären, oralen, intraperitonealen, inhalativen und topischen Darreichungsform formuliert.

Entsprechend der Verabreichungsart liegt die erfindungsgemäße Verbindung, insbesondere Phenyl-Amino-Ethan-Hydrazid bzw. dessen Derivate, vorzugsweise in Form einer Infusion, Tablette, Kapsel, Creme, Gel, Emulsion oder eines Pflasters vor.

Je nach Darreichungsform umfasst die erfindungsgemäße pharmazeutische Zusammensetzung neben den erfindungsgemäßen Verbindungen Hilfsstoffe, wie z.B. Sprengmittel und Stabilisatoren, Trägerstoffe und Verdünnungsmittel.

Beispiele für übliche Hilfsstoffe, Trägerstoffe und Verdünnungsmittel sind Gelatine, natürliche Zucker (wie Rohrzucker oder Milchzucker, Lecithin, Pektin, Stärke (z.B. Maisstärke) sowie Stärkederivate, Cyclodextrine und Cyclodextrinderivate, Polyvinylpyrrolidon, Gelatine, Gummi arabicum, Alginsäure, Tylose, Talkum, Lycopodium, Kieselsäure (z.B. kolloidale), Lävulose, Traganth, Natriumchlorid, Stearate, Magnesium- und Calciumsalze von Fettsäuren mit 12 bis 22 C-Atomen, insbesondere der gesättigten (z.B. Stearate), Polyethylenglycol mit einem mittleren Molekulargewicht zwischen 200 und 20.000, vorzugsweise zwischen 200 und 5.000, insbesondere zwischen 200 und 1.000, oder deren Gemische und/oder Polymerisate aus Vinylpyrrolidon und/oder Mischpolymerisate aus Vinylpyrrolidon und Vinylacetat. Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglycol, Pentaerythrit, Sorbit, Mannit usw., die gegebenenfalls auch verethert sein können, Benzylbenzoat, Dioxolane, Glyzerinformale, Tetrahydrofurfurylalkohol, Polyglykolether mit C₁- bis C₁₂-Alkoholen, Dimethylacetamid, Lactamide, Lactate, Ethylcarbonate, Silicone (insbesonder mittelviskose Polydimethylsiloxane), Calciumcarbonat, Natriumcarbonat, Calciumphosphat, Natriumphosphat, Magnesiumcarbonat, Gummi arabicum, Alginsäure, Stearate, Fette und ähnlich wirkende Stoffe. Bei Lösungen, wie z.B. Infusionen, können verschiedene Puffersysteme eingesetzt werden.

Ein weiterer Aspekt der vorliegenden Erfindung betrifft eine pharmazeutische Zusammensetzung umfassend zumindest eine Verbindung wie hierin beschrieben zur Verwendung zur Behandlung und/oder Prävention von inflammatorischen Erkrankungen, welche in Zusammenhang mit Eosinophiler Peroxidase stehen, und wobei die inflammatorische Erkrankung ausgewählt ist aus der Gruppe bestehend aus Asthma bronchiale, cystische Fibrose, Rhinitis, Sinusitis und Churg-Strauss Syndrom.

Die erfindungsgemäße pharmazeutische Zusammensetzung liegt vorzugsweise in Form einer Infusion, Tablette, Kapsel, Creme, Gel, Emulsion oder eines Pflasters vor.

Ein noch weiterer Aspekt der vorliegenden Erfindung betrifft die Verwendung der Verbindungen gemäß der vorliegenden Erfindung zur Herstellung eines Medikaments zur Behandlung und/oder Prävention von inflammatorischen Erkrankungen, welche in Zusammenhang mit Eosinophiler Peroxidase stehen, und wobei die inflammatorische Erkrankung ausgewählt ist aus der Gruppe bestehend aus Asthma bronchiale, cystische Fibrose, Rhinitis, Sinusitis und Churg-Strauss Syndrom.

Ein weiterer Aspekt der vorliegenden Erfindung betrifft ein Verfahren zur Behandlung und/oder Prävention von inflammatorischen Erkrankungen, welche in Zusammenhang mit Eosinophiler Peroxidase stehen, und wobei die inflammatorische Erkrankung ausgewählt ist aus der Gruppe bestehend aus Asthma bronchiale, cystische Fibrose, Rhinitis, Sinusitis und Churg-Strauss Syndrom, durch Verabreichung einer oder mehrerer der erfindungsgemäßen Verbindungen.

Die vorliegende Erfindung wird anhand der folgenden Beispiele näher erläutert, ohne jedoch auf diese beschränkt zu sein.

### BEISPIELE:

### Beispiel 1:

Um zu testen, inwieweit die erfindungsgemäßen Substanzen in der Lage sind EPO zu inhibieren, wurden die Substanzen auf ihr inhibitorisches Potential getestet. Dabei wurde der IC₅₀-Wert als vergleichbarer Parameter bestimmt. IC₅₀ ist dabei jene Inhibitor-Konzentration, die benötigt wird, um ein Enzym, hier EPO, zu 50 % zu inhibieren. Diese Konzentration wird UV/visspektrophotometrisch bei 290 nm im Steady-state mit einem Monochlorodimedon-MCD-Assay bestimmt.

### Bestimmung der inhibitorischen Wirkung

### IC₅₀-Werte Bestimmung

Eosinophile Peroxidase bildet eine Vielzahl von unterschiedlichen Enzym-Intermediaten aus und ist in der Lage, eine große Anzahl von Redox-Reaktionen zu katalysieren. Die physiologische Rolle von EPO ist die Oxidation von Bromid bzw. Thiocyanat zu hypobromiger Säure bzw. Hypothiocyante (auch als Halogenierungs-Zyklus bezeichnet). Und genau diese Reaktion gilt es auch zu inhibieren. In Gegenwart phenolischer Substanzen kann das Enzym aber auch den sogenannten Peroxidase-Zyklus durchlaufen.

Um die inhibierenden Eigenschaften der erfindungsgemäßen Substanzen zu bestimmen, wurde eine Methode verwendet bei der die Bromierungsaktivität untersucht wird.

### Bromierungs-Aktivität

Das Ausmaß der Inhibierung der physiologischen Bromid-Oxidation wurde photometrisch mit Monochlorodimedon bestimmt. Die Halogenierungsrate (Anfangssteigung der Kurve bei 290 nm) mit Inhibitor wurde in Relation zu einem Blindwert (ohne Inhibitor) gesetzt und daraus die Inaktivierungsrate (in %) ermittelt. Diese wurde in einem Diagramm (y-Achse) gegenüber der Inhibitorkonzentration (x-Achse) aufgetragen und aus dem hyperbolischen Fit der Kurve der IC₅₀-Wert für jeden Inhibitor ermittelt.
100 mM Phosphatpuffer, pH 7.0
100 µM Monochlorodimedon
100 mM Bromid
20 nM EPO
100 µM HOOH
0,001 - 500 µM Inhibitor

### Phenyl-Amino-Ethanhydrazide

Bei der Untersuchung verschiedener Substanzgruppen, die auf Grund ihrer Struktur voraussichtlich in das katalytische Zentrum der EPO (und der homologen LPO) passen, und dort auch die Aktivität hemmen, hat sich herausgestellt, dass die Substanzgruppe der Phenyl-Amino-Ethanhydrazide (III), besonders aber deren Derivate und halogenierten Derivate davon, sehr gute selektive Inhibitoren der EPO sind. Beispiele für entsprechende Derivate, vor allem aber halogenierte Derivate, sind wie folgt zu nennen: Tabelle 2 zeigt an Hand mehrerer Beispiele die Selektivität der Phenyl-Amino-Ethanhydrazide für EPO (und auch für die homologe LPO), nicht aber für MPO:

**Tabelle 2: Beispiel für Phenyl-Amino-Ethanhydrazid Derivate und das inhibitorische Potential (IC₅₀: Konzentration, bei der 50 % der Enzymaktiviät inhibiert sind)**

| Phenyl-Amino-Ethan-Hydrazide (PAEH's) | Strukturformel | EPO+Br [µM] | LPO+Br [µM] | MPO+Br [µM] | MPO+Cl [µM] |
|---|---|---|---|---|---|
| (1) | | | | | |
| | 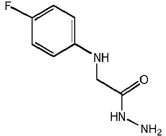 | **0,240** | **0,540** | **4,120** | **5,430** |
| **4-Fluoro-** | | | | | |
| Phenylaminoethan-Hydrazid | | | | | |
| (2) | 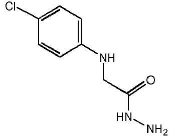 | **0,024** | **0,030** | **1,200** | **1,970** |
| **4-Chloro** | | | | | |
| Phenylaminoethan-Hydrazid | | | | | |
| (3) | 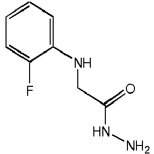 | **0,009** | **0,100** | **1,900** | **8,800** |
| **2-Fluoro** | | | | | |
| Phenylaminoethan-Hydrazid | | | | | |
| (4) | 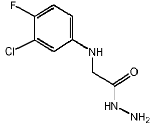 | **0,019** | **0,140** | **0,547** | **2,400** |
| **4-Fluoro** | | | | | |
| **3-Chloro** | | | | | |
| Phenylaminoethan-Hydrazid | | | | | |
| (5) | 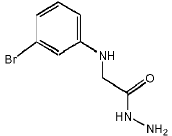 | **0,017** | **0,040** | **1,600** | **3,700** |
| **3-Bromo** | | | | | |
| Phenylaminoethan-Hydrazid | | | | | |
| (6) | 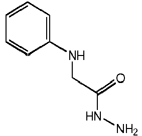 | **2,290** | **4,967** | **84,56** | **46,04** |
| Nicht **halogeniertes** | | | | | |
| Phenylaminoethan-Hydrazid | | | | | |
| (7) | 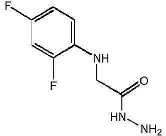 | **0,034** | **0,322** | **2,040** | **6,550** |
| **2,4-di-Fluoro** | | | | | |
| Phenylaminoethan-Hydrazid | | | | | |
| (8) | 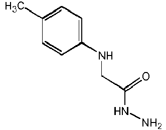 | **2,270** | **2,773** | **29,40** | **32,19** |
| **Nicht halogeniertes** | | | | | |
| 4-Methyl | | | | | |
| Phenylaminoethanhydrazid | | | | | |

Die Verbindung (3) 2-Fluorophenyl-NH-Ethanhydrazid hat einen IC₅₀-Wert für EPO von 0,009 µM, aber für MPO einen wesentlich höheren IC₅₀-Wert von 1,900 bzw. 8,800 µM. Das heißt, diese Substanz stellt für EPO einen sehr guten Inhibitor dar, nicht aber für MPO aus der gleichen Enzymfamilie der humanen Peroxidasen.

Weiters kann man aus Tabelle 2 entnehmen, dass halogenierte Phenyl-Amino-Ethanhydrazid Derivate stärker inhibieren als nicht halogenierte.

Verbindung (6) Phenyl-Amino-Ethanhydrazid zeigt einen IC₅₀-Wert von 2,290 µM. Dieses Potential kann bereits bei guter Verträglichkeit als Inhibitor zur therapeutischen Anwendung führen. Jedoch zeigt Beispiel Nummer (3) 2-Fluorophenyl-NH-Ethanhydrazid mehr als das 200fache Potential mit einem IC₅₀-Wert von 0,009 µM. Dadurch sind sehr geringe therapeutische Konzentrationen möglich, die dadurch auch eventuell auftretende unerwünschte Nebenwirkungen minimieren.

### Referenzbeispiel 2:

In einer weiteren Testreihe wurde untersucht, inwieweit weitere Substanzen mit der allgemeinen Formel (I) in der Lage sind die Aktivität von EPO zu inhibieren. Als Beispiel wurde Isoniazid (Pyridin-4-carbohydrazid) herangezogen, bei der Rₓ in der allgemeinen Formel (I) einen Pyridin-Rest darstellt. Die Versuche wurden wie in Beispiel 1 dargestellt durchgeführt.

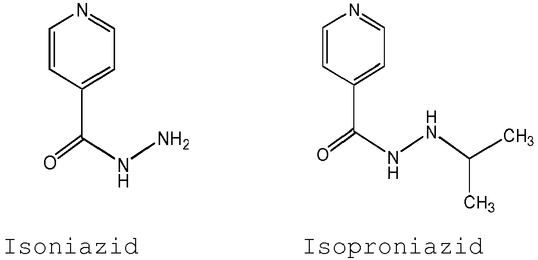

Es wurde festgestellt, dass Isoniazid einen IC₅₀-Wert von 6,04 µM aufweist.

Um zu den Einfluss der freien Aminogruppe am Hydrazid-Rest der allgemeinen Formel (I) auf die inhibitorischen Eigenschaften der erfindungsgemäßen Substanzen auf EPO zu untersuchen, wurde ein Derivat von Isoniazid, nämlich N'-Isopropylisonicotinohydrazid (Iproniazid), untersucht. Dabei wurde überraschender Weise festgestellt, dass Iproniazid einen IC₅₀-Wert von über 500 µM aufweist.

Dies belegt, dass für die starke Inhibierung der EPO Aktivität neben anderen Eigenschaften (II) jedenfalls die freie Aminogruppe der Substanzen gemäß der allgemeinen Formel (I) maßgeblich ist. Am Beispiel der strukturverwandten Substanzen Isoniazid und Iproniazid konnte dies eindrucksvoll gezeigt werden. Die Derivatisierung der freien Aminogruppen führt zu einem Verlust der Hemmstärke.

### Beispiel 3:

In einer weiteren Testreihe, welche nach demselben Protokoll durchgeführt wurde in Beispiel 1 angeführt, wurden weitere erfindungsgemäße Verbindungen auf deren Fähigkeiten eosinophile Peroxidase zu inhibieren untersucht. Die Ergebnisse dieser Versuche und die darin verwendeten Verbindungen sind der folgenden Tabelle zu entnehmen.

| **#** | **Name** | **MG** | **CAS** | **IC50 EPO/Br (µM)** |
|---|---|---|---|---|
| PD01 | N-(2-Fluorophenyl)Glycin | 169,155423 | 5319-42-6 | **1,0** |
| | 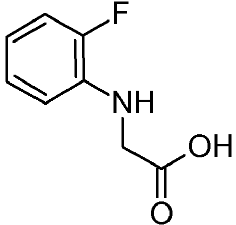 | | | |
| PD02 | 2-[(4-chlorophenyl)amino]Essigsäure | 185,61146 | 5465-90-7 | **0,2** |
| | 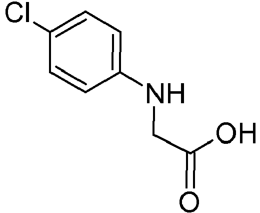 | | | |

### Beispiel 4:

Um die pharmakologische Wirkung der erfindungsgemäßen Verbindungen zu zeigen können Tiermodelle herangezogen werden. Mithilfe von Tiermodellen ist es möglich experimentell zu belegen inwieweit pharmakologisch aktive Wirkstoffe entsprechende Wirkung aufweisen.

### 1. Asthma bronchiale

Für die Ausprägung und Fortschritt von Asthma bronchiale sind mehrere Faktoren verantwortlich (1): Allergene, emotionaler Stress, körperliche Belastung, kalte Luft und alle Kombinationen dieser Faktoren. Die pathophysiologische Antwort ist sehr komplex, aber es zeigt sich ein "roter Faden" zu unserem Target, der EPO. T-helper 2 (Th2) Zellen führen zur Interleukin Ausschüttung, besonders IL-5, das die Freisetzung von Eotaxinen hervorruft. Diese führen zur Migration von Eosinophilen Granulozyten an den Lungenwirkort. Die bei der Allergie erhöhten IgE Spiegel und IgE Rezeptoren an den Eosinophilen Zellen führen zur Degranulation und zur Freisetzung von Proteinen mit einem 60 % Anteil von EPO. Die EPO katalysiert die Oxidation von Halogeniden und Thiocyanat, wobei hochreaktive Oxidationsprodukte entstehen, die zur Abwehr von Parasiten und Mikroorganismen freigesetzt werden, aber (im Fall von Asthma und anderen chronischen Erkrankungen) auch gewebszerstörend wirken.

Es wird daher ein "chronisches Modell" benötigt, wobei nachgewiesen werden muss, ob dieser Mechanismus auch abläuft und an die humanen Abläufe angenähert ist. Mit diesem Modell kann dann die Wirkung von EPO Inhibtoren getestet werden.

Zum Nachweis der Wirkung werden entsprechende **Tiermodelle** herangezogen, die jedoch gerade im Zusammenhang mit Asthma und EPO komplex sind.

### Vorgehensweise:

Balb/c Mäuse mit einem Körpergewicht von 18-21 g werden in eine Akklimatisierungsphase von einer Woche gehalten.

Die Irrelevanz von Ovalbumin und Induzierung von Asthma (allergische Atemwegsentzündung) ist bekannt, deshalb wird mit Hausstaubmilben oder Graspollen stimuliert. Über 7 Wochen lang wird täglich das Allergen intranasal appliziert. Diese Stimulierung resultiert direkt in eine Asthma Symptomatik mit AHR (acute airway hyperresponsivesnes) und eosinophiler Atemwegsentzündung (Johnson et al. 2004, Am J Respir Crit Care Med 169:378-385; Johnson et al. 2008, Am J Physiol lung Cell Mol Physiol 295:L780-L788).

Mittels ELISA werden schließlich Entzündungsparameter, Eosinophile Granulozyten und EPO im BALB (bronchioalveolare Flüssigkeit) Überstand gemessen. Wenn EPO aktiv ist, dann werden diese Individuen zur Therapie und zur Kontrollgruppe aufgeteilt. Die Therapiegruppe erhält die erfindungsgemäßen Verbindungen (1 - 10 mg/kg KG täglich), während die Kontrollgruppe ein Placebo bekommt. Als Parameter für die Entwicklung der Allergie und chronischen Entzündung der Atemwege und Lunge wird unter anderem die Anzahl der Exazerbationen (schwerer Anfall) und das Ausmaß der AHR herangezogen. Eine dritte Gruppe kann mit Dexamethason (u. anderen) auf herkömmliche Weise therapiert werden.

### 2. Rhinitis und Sinusitis

Die Wirkungen der erfindungsgemäßen Verbindungen bei Erkrankungen der Nebenhöhlen und Siebbeine können mit demselben Tiermodell wie Asthma bronchiale festgestellt werden.

### 3. Cystische Fibrose

Einfach durchführbarer Test mit Mäusen. Da die zystische Fibrose auch mit Infektion verbunden ist, werden die Versuchstiere infiziert und nach Ausbruch der Erkrankung behandelt (Arzneimittelkandidat - Placebo - konventionell) (Wang Y, et al. Respir Res. 2010 Nov 30;11:166; Guilbault C, et al. Lab Anim. 2005 Jul; 39 (3) :336-52).

## Patentansprüche

1. Verbindung mit der allgemeinen Formel (III): 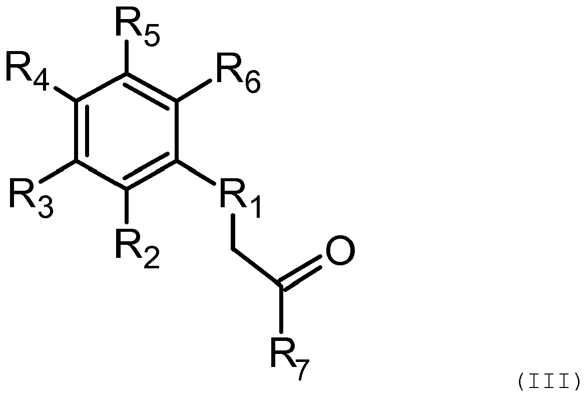 zur Verwendung bei der Behandlung und/oder Prävention von inflammatorischen Erkrankungen, welche in Zusammenhang mit Eosinophiler Peroxidase stehen, wobei
R₁ NH ist,
R₂, R₃, R₄, R₅ und R₆ unabhängig voneinander H, OH, F, Cl, Br, I oder eine C₁ bis C₅ Alkylgruppe sind und
R₇ H, OH, NH₂, NH-NH₂ oder CH₃ ist, und wobei die inflammatorische Erkrankung ausgewählt ist aus der Gruppe bestehend aus Asthma bronchiale, cystische Fibrose, Rhinitis, Sinusitis und Churg-Strauss Syndrom.

2. Verbindung zur Verwendung nach Anspruch 1, **dadurch gekennzeichnet, dass** R₇ NH-NH₂ ist und die Verbindung die allgemeine Formel (IV) 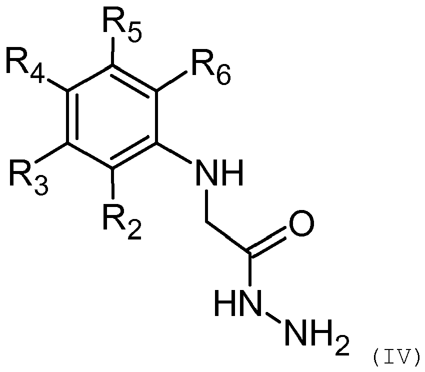 aufweist.

3. Verbindung zur Verwendung gemäß Anspruch 1 oder 2, **dadurch gekennzeichnet, dass** die C₁ bis C₅ Alkylgruppe ausgewählt ist aus der Gruppe bestehend aus CH₃ und CH₂CH₃.

4. Verbindung zur Verwendung gemäß einem der Ansprüche 1 bis 3, **dadurch gekennzeichnet, dass** R₁ NH, R₂ F oder H, R₃ Cl, Br oder H, R₄ Cl, F, CH₃ oder H, R₅ und R₆ H und R₇ OH oder NH-NH₂ ist.

5. Verbindung zur Verwendung gemäß einem der Ansprüche 1 bis 4, **dadurch gekennzeichnet, dass** die Verbindung ausgewählt ist aus der Gruppe bestehend aus 2-Fluoro-Phenylaminoethan-Hydrazid, 4-Fluoro-Phenylaminoethan-Hydrazid, 2,4-di-Fluoro-Phenylaminoethan-Hydrazid, 4-Chloro-Phenylaminoethan-Hydrazid, 3-Chloro-4-Fluoro-Phenylaminoethan-Hydrazid, 3-Bromo-4-Fluoro-Phenylaminoethan-Hydrazid, 4-Methyl-Phenylaminoethan-Hydrazid, Phenylaminoethan-Hydrazid, N-(2-Fluorophenyl)Glycin und 2-[(4-chlorophenyl)amino]Essigsäure.

6. Verbindung zur Verwendung gemäß einem der Ansprüche 1 bis 5, **dadurch gekennzeichnet, dass** die Verbindung in einer intravenösen, intrakavitären, oralen, intraperitonealen, inhalativen und topischen Darreichungsform vorgesehen ist.

7. Verbindung zur Verwendung gemäß einem der Ansprüche 1 bis 6, **dadurch gekennzeichnet, dass** die Verbindung in Form einer Infusion, Tablette, Kapsel, Creme, Gel, Emulsion oder eines Pflasters vorliegt.

8. Verbindung zur Verwendung gemäß einem der Ansprüche 1 bis 7, **dadurch gekennzeichnet, dass** die Verbindung in einer Menge von 0,01 bis 2.000 mg/kg Körpergewicht, vorzugsweise 0,1 bis 1.000 mg/kg Körpergewicht, noch mehr bevorzugt 0,1 bis 500 mg/kg Körpergewicht, verabreicht wird.

9. Pharmazeutische Zusammensetzung umfassend zumindest eine Verbindung gemäß einem der Ansprüche 1 bis 5, zur Verwendung zur Behandlung und/oder Prävention von inflammatorischen Erkrankungen, welche in Zusammenhang mit Eosinophiler Peroxidase stehen, wobei die inflammatorische Erkrankung ausgewählt ist aus der Gruppe bestehend aus Asthma bronchiale, cystische Fibrose, Rhinitis, Sinusitis und Churg-Strauss Syndrom.

10. Pharmazeutische Zusammensetzung zur Verwendung gemäß Anspruch 9, **dadurch gekennzeichnet, dass** die Verbindung in einer intravenösen, intrakavitären, oralen, intraperitonealen, inhalativen und topischen Darreichungsform vorgesehen ist.

11. Pharmazeutische Zusammensetzung zur Verwendung gemäß Anspruch 9 oder 10, **dadurch gekennzeichnet, dass** die Verbindung in Form einer Infusion, Tablette, Kapsel, Creme, Gel, Emulsion oder eines Pflasters vorliegt.

12. Verwendung einer Verbindung gemäß einem der Ansprüche 1 bis 5 zur Inhibierung eosinophiler Peroxidase in vitro.

## Claims

1. A compound of the general formula (III): 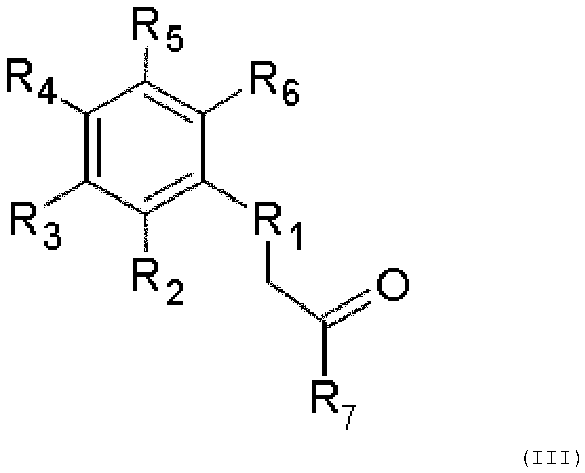 for use in the treatment and/or prevention of inflammatory diseases, which are associated with eosinophil peroxidase, wherein R₁ is NH,
R₂, R₃, R₄, R₅ and R₆, independent of one another, are H, OH, F, Cl, Br, I or a C₁ to C₅ alkyl group, and
R₇ is H, OH, NH₂, NH-NH₂ or CH₃, and wherein
said inflammatory disease is selected from the group consisting of bronchial asthma, cystic fibrosis, rhinitis, sinusitis, and Churg-Strauss syndrome.

2. The compound for use according to claim 1, **characterized in that** R₇ is NH-NH₂ and said compound has the general formula(IV).

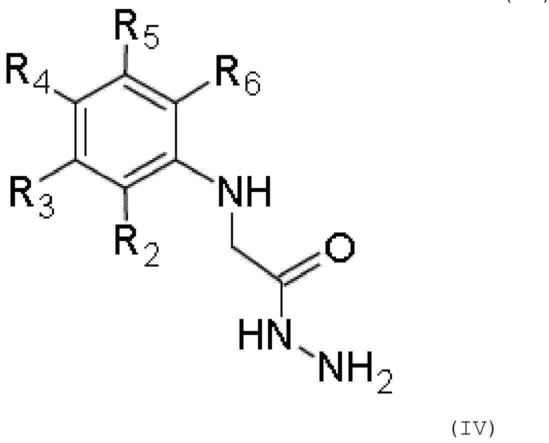

3. The compound for use according to claims 1 or 2, **characterized in that** said C₁ to C₅ alkyl group is selected from the group consisting of CH₃ and CH₂CH₃.

4. The compound for use according to any of claims 1 to 3, **characterized in that** R₁ is NH, R₂ is F or H, R₃ is Cl, Br or H, R₄ is Cl, F, CH₃ or H, R₅ and R₆ are H, and R₇ is OH or NH-NH₂.

5. The compound for use according to any of claims 1 to 4, **characterized in that** said compound is selected from the group consisting of 2-fluoro-phenylaminoethane-hydrazide, 4-fluoro-phenylaminoethane-hydrazide, 2,4-di-fluoro-phenylaminoethane-hydrazide, 4-chloro-phenylaminoethane-hydrazide, 3-chloro-4-fluoro-phenylaminoethane-hydrazide, 3-bromo-4-fluoro-phenylaminoethane-hydrazide, 4-methyl-phenylaminoethane-hydrazide, phenylaminoethane-hydrazide, N-(2-fluorophenyl)glycine, and 2-[(4-chlorophenyl)amino]acetic acid.

6. The compound for use according to any of claims 1 to 5, **characterized in that** said compound is provided in an intravenous, intracavitary, oral, intraperitoneal, inhalation and topical dosage form.

7. The compound for use according to any of claims 1 to 6, **characterized in that** said compound is present in the form of an infusion, tablet, capsule, cream, gel, emulsion, or patch.

8. The compound for use according to any of claims 1 to 7, **characterized in that** said compound is administered in an amount of 0.01 to 2,000 mg/kg of body weight, preferably 0.1 to 1,000 mg/kg of body weight, still more preferred 0.1 to 500 mg/kg of body weight.

9. A pharmaceutical composition comprising at least one compound according to any of claims 1 to 5, for use in the treatment and/or prevention of inflammatory diseases, which are associated with eosinophil peroxidase, wherein said inflammatory disease is selected from the group consisting of bronchial asthma, cystic fibrosis, rhinitis, sinusitis, and Churg-Strauss syndrome.

10. The pharmaceutical composition for use according to claim 9, **characterized in that** said compound is provided in an intravenous, intracavitary, oral, intraperitoneal, inhalation and topical dosage form.

11. The pharmaceutical composition for use according to claims 9 or 10, **characterized in that** said compound is present in the form of an infusion, tablet, capsule, cream, gel, emulsion or patch.

12. Use of a compound according to any of claims 1 to 5 for the inhibition of eosinophil peroxidase in vitro.

## Revendications

1. Composé de formule générale (III) : 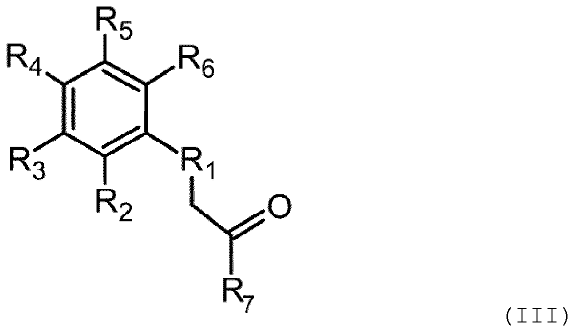 pour son utilisation dans le traitement et/ou la prévention de maladies inflammatoires qui sont associées à la peroxydase éosinophile, dans lequel
R₁ est NH,
R₂, R₃, R₄, R₅ et R₆ sont, indépendamment les uns des autres, H, OH, F, Cl, Br, I ou un groupe alkyle en C₁ à C₅ et
R₇ est H, OH, NH₂, NH-NH₂ ou CH₃, et dans lequel
la maladie inflammatoire est choisie dans le groupe consistant en l'asthme bronchique, la fibrose kystique, la rhinite, la sinusite et le syndrome de Churg-Strauss.

2. Composé pour son utilisation selon la revendication 1, **caractérisé en ce que** R₇ est NH-NH₂ et le composé présente la formule générale (IV)

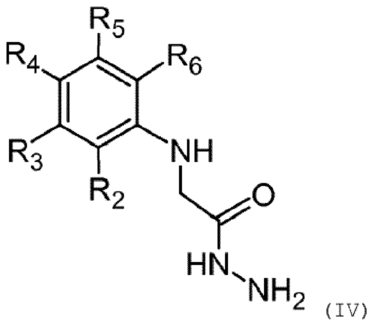

3. Composé pour son utilisation selon la revendication 1 ou 2, **caractérisé en ce que** le groupe alkyle en C₁ à C₅ est choisi dans le groupe consistant en CH₃ et CH₂CH₃.

4. Composé pour son utilisation selon l'une des revendications 1 à 3, **caractérisé en ce que** R₁ est NH, R₂ est F ou H, R₃ est Cl, Br ou H, R₄ est Cl, F, CH₃ ou H, R₅ et R₆ sont H et R₇ est OH ou NH-NH₂.

5. Composé pour son utilisation selon l'une des revendications 1 à 4, **caractérisé en ce que** le composé est choisi dans le groupe consistant en l'hydrazide de 2-fluoro-phénylaminoéthane, l'hydrazide de 4-fluoro-phénylaminoéthane, l'hydrazide de 2,4-di-fluorophényl-aminoéthane, l'hydrazide de 4-chloro-phénylaminoéthane, l'hydrazide de 3-chloro-4-fluoro-phénylaminoéthane, l'hydrazide de 3-bromo-4-fluoro-phénylaminoéthane, l'hydrazide de 4-méthyl-phénylaminoéthane, l'hydrazide de phénylaminoéthane, la N-(2-fluorophényl)glycine et l'acide 2-[(4-chlorophényl)amino]acétique.

6. Composé pour son utilisation selon l'une des revendications 1 à 5, **caractérisé en ce que** le composé est prévu sous une forme galénique intraveineuse, intracavitaire, orale, intrapéritonéale, pour inhalation et topique.

7. Composé pour son utilisation selon l'une des revendications 1 à 6, **caractérisé en ce que** le composé se présente sous la forme d'une perfusion, d'un comprimé, d'une gélule, d'une crème, d'un gel, d'une émulsion ou d'un patch.

8. Composé pour son utilisation selon l'une des revendications 1 à 7, **caractérisé en ce que** le composé est administré en une quantité de 0,01 à 2 000 mg/kg de poids corporel, de préférence de 0,1 à 1 000 mg/kg de poids corporel, de manière encore davantage préférée de 0,1 à 500 mg/kg de poids corporel.

9. Composition pharmaceutique, comprenant au moins un composé selon l'une des revendications 1 à 5, pour son utilisation pour le traitement et/ou la prévention de maladies inflammatoires qui sont associées à la peroxydase éosinophile, dans laquelle la maladie inflammatoire est choisie dans le groupe consistant en l'asthme bronchique, la fibrose kystique, la rhinite, la sinusite et le syndrome de Churg-Strauss.

10. Composition pharmaceutique, pour son utilisation selon la revendication 9, **caractérisée en ce que** le composé est prévu sous une forme galénique intraveineuse, intracavitaire, orale, intrapéritonéale, pour inhalation et topique.

11. Composition pharmaceutique, pour son utilisation selon la revendication 9 ou 10, **caractérisée en ce que** le composé se présente sous la forme d'une perfusion, d'un comprimé, d'une gélule, d'une crème, d'un gel, d'une émulsion ou d'un patch.

12. Utilisation d'une composition selon l'une des revendications 1 à 5, pour inhiber la peroxydase éosinophile *in vitro.*
